(19) **Europäisches Patentamt** / **European Patent Office** / **Office européen des brevets**

(11) **EP 2 819 573 B1**

(12) **EUROPEAN PATENT SPECIFICATION**

(45) Date of publication and mention of the grant of the patent:
**05.07.2017 Bulletin 2017/27**

(21) Application number: **13706529.8**

(22) Date of filing: **27.02.2013**

(51) Int Cl.:
*A61M 25/10* (2013.01)     *A61B 5/026* (2006.01)
*A61B 5/0295* (2006.01)     *A61B 5/00* (2006.01)
*A61B 5/1459* (2006.01)     *A61B 5/03* (2006.01)
*A61B 5/0456* (2006.01)

(86) International application number:
**PCT/EP2013/053878**

(87) International publication number:
**WO 2013/127819 (06.09.2013 Gazette 2013/36)**

(54) **APPARATUS AND METHOD FOR MEASURING BLOOD FLOW WITHIN THE GASTROINTESTINAL TRACT**

VORRICHTUNG UND VERFAHREN ZUR MESSUNG DES BLUTFLUSSES IM MAGEN-DARM-TRAKT

APPAREIL ET PROCÉDÉ DE MESURE DE DÉBIT SANGUIN À L'INTÉRIEUR DU TRACTUS GASTRO-INTESTINAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2012 US 201213406297**

(43) Date of publication of application:
**07.01.2015 Bulletin 2015/02**

(73) Proprietor: **APD Advanced Perfusion Diagnostics 69008 Lyon (FR)**

(72) Inventor: **BEUTE, Jan NL-1355 HK Almere (NL)**

(74) Representative: **Lemoine, Jean-Sébastien et al Cabinet BREV&SUD 55, avenue Clément Ader 34170 Castelnau-le-Lez (FR)**

(56) References cited:
WO-A1-94/21163     WO-A1-96/25090
US-A- 5 743 261     US-A- 5 987 351
US-A1- 2008 319 339     US-A1- 2009 281 399
US-A1- 2010 312 128     US-B1- 6 741 884

**Description**

## II. Field Of The Invention

This application generally relates to apparatus and methods for measuring blood flow within an organ and/or body part.

## III. Background Of The Invention

Plethysmography is a method for measuring changes in volume within an organ and/or body parts, generally resulting from fluctuations in blood flow or air therein. Blood flow fluctuations within the gastrointestinal tract may indicate serious medical conditions such as circulatory shock, commonly known as shock. When a patient experiences shock, the body redistributes blood flow to the vital organs such as the brain, heart, and muscles, causing a reduced blood flow in other organs, including organs in the gastrointestinal tract. Shock may cause significant deterioration in the function of the gastrointestinal tract leading to further deterioration in the general physical condition of the patient and even death.
Previously known methods for measuring blood flow of the gastrointestinal tract include indocyanine green clearing, stomach tonometry, videoscopy of the tongue blood flow, and determination of the oxygen saturation of the large intestine. These methods suffer from a variety of drawbacks including delayed measurement results that often must be obtained after some time in a laboratory. Additionally, the previously known methods are not specific with respect to the measurement of local blood flow within the intestine and are believed to be relatively unreliable.
U.S. Patent Publication No. 2008/0319339 to Beute describes a balloon catheter coupled to monitoring device for measuring blood flow within the gastrointestinal tract. The catheter includes a sensor disposed on the balloon to produce a signal corresponding to pressure or pressure changes.
U.S. Patent No. 7,618,376 to Kimball describes a device for assessing the degree of systemic perfusion in a patient, and includes a Doppler blood flow sensor configured for placement in the upper gastrointestinal tract and a blood pressure monitor. The device requires the measurement of blood pressure for assessing the degree of systemic perfusion.
U.S. Patent No. 5,743,261 to Mainiero describes a blood flow measurement system according to the preamble of claim 1.
In view of the foregoing, it would be desirable to provide a system, and methods of using the same, for non-invasively measuring blood flow within the gastrointestinal tract accurately and in real time.

## IV. Summary Of The Invention

The object of the present invention is achieved with a system and method according to the independent claims. The present invention overcomes the drawbacks of previously-known systems by providing a blood flow measurement system for measuring gastrointestinal blood flow that may be used as an indication of circulatory, septic, and hypovolemic shock, as well as other situations resulting in hypoperfusion of the gastrointestinal tract. The blood flow measurement may include measurements indicating the amount of red blood cells carrying oxygen in the blood to ensure tissue oxygenation and prevent ischemia resulting in tissue/organ damage. The blood flow measurement system of the present invention includes a catheter and a processor. The catheter is configured for placement within a gastrointestinal tract of a patient and includes a distal end, a proximal end, an expandable member disposed near the distal end, a lumen extending between the proximal end and the expandable member, and an optical sensor disposed adjacent to the expandable member and configured to generate a signal indicative of blood flow within the gastrointestinal tract. The processor is operatively coupled to the optical sensor and the expandable member. The processor may be configured to control the optical sensor and to receive the signal from the optical sensor. The processor may be further configured to control periodic inflation and deflation of the expandable member.
A pump may be operatively coupled to the expandable member through the lumen and to the processor, and the processor may be configured to cause pump to periodically inflate and deflate the expandable member. In one embodiment, the blood measurement system further includes a housing configured to house the processor and the pump. The system also may have a valve operatively coupled to the pump and configured to control gas flow to the expandable member.
The optical sensor may include a diode and a photodiode. The diode may be configured to emit light into the gastrointestinal tract and the photodiode may be configured to receive the light reflected from the gastrointestinal tract. The optical sensor may be disposed within or outside the expandable member.
Preferably, the blood flow measurement system includes measurement software configured to run on a computer operatively coupled to the processor. The measurement software may be configured to process the signal to calculate an area indicative of a blood flow rate, e.g., perfusion rate, within the gastrointestinal tract, e.g., capillary blood flow to the intestinal lining.

The system also may include an electrocardiogram (ECG) lead assembly operatively coupled to the processor and configured to sense an ECG signal based on electrical activity of a heart of the patient. The measurement software may determine an R-wave signal based on the ECG signal.

The blood flow measurement system further may include a photoplethysmogram (PPG) module operatively coupled to the processor and the optical sensor. The PPG module may be configured to receive the signal from the optical sensor and to generate a PPG signal based on the signal and to transmit the PPG signal to the processor. The measurement software may receive the PPG signal and to determine a PPG segment based on the R-wave signal. The measurement software is configured to calculate the area under the PPG segment for calculating the blood flow rate within the gastrointestinal tract.

The blood flow measurement system of the present invention provides non-invasive measurement of gut perfusion and/or gastrointestinal blood flow rate and may be used, for example, on patients in an intensive care unit with a risk of shock as well as high risk surgical patients such as patients undergoing thoracic and/or abdominal surgery.

In accordance with one aspect of the present invention, a method for measuring blood flow within a gastrointestinal tract of a patient is provided. The method may include introducing an optical sensor into the gastrointestinal tract, generating a signal indicative of blood flow within the gastrointestinal tract using the optical sensor, processing the signal to generate a PPG signal, generating an ECG signal based electrical activity of a heart of the patient, determining a PPG segment of the PPG signal based on the ECG signal, and measuring blood flow, e.g., gastrointestinal perfusion, within the gastrointestinal tract based on the PPG segment.

The optical sensor may be introduced on an expandable member having the optical sensor disposed thereon and the expandable member may be disposed on a catheter.

The PPG segment may be determined by determining a starting point and an ending point of the PPG signal based on the ECG signal. The blood flow within the gastrointestinal tract may be measured based on an area below the PPG signal and above a boundary line between the starting point and the ending point. The PPG segment of the PPG signal may be determined based on an R-wave of the ECG signal.

## V. Brief Description Of The Drawings

FIG. 1 depicts the components of an exemplary blood flow measurement system constructed in accordance with the principles of the present invention.

FIGS. 2A and 2B are perspective views of a distal end of an exemplary catheter suitable for use with the system of the present invention, in which FIG. 2B corresponds to detail region 2B of FIG. 2A.

FIGS. 2C and 2D are, respectively, side and sectional views of the distal end of the exemplary catheter of FIGS. 2A and 2B, in which FIG. 2D is a sectional view of line 2D-2D of FIG. 2C.

FIG. 3 is a schematic diagram of the electrical and pumping components disposed within a housing in accordance with an exemplary embodiment of the present invention.

FIG. 4 illustrates an exemplary method for measuring blood flow in accordance with the principles of the present invention.

FIG. 5 illustrates an exemplary graph showing an ECG signal having determined R-waves marked thereon.

FIG. 6 depicts an exemplary graphic of an ECG signal and a PPG signal for determining a starting point of a PPG segment in accordance with the principles of the present invention.

FIG. 7 illustrates an exemplary graph displaying an AC area and a DC area of a PPG segment used for measuring blood flow in accordance with the principles of the present invention.

FIG. 8A illustrates an alternative catheter for use in a blood flow measurement system constructed not in accordance with the principles of the present invention.

FIGS. 8B and 8C are, respectively, perspective and side views of the distal end of the alternative catheter of FIG. 8A.

FIGS. 9A and 9B are perspective views of a distal end of another alternative catheter for use in a blood flow measurement system constructed not in accordance with the principles of the present invention, in which FIG. 9A shows the expandable member in a contracted state and FIG. 9B shows the expandable member in an expanded state.

FIG. 9C is a side view of the alternative catheter shown in FIG. 9B.

FIG. 10A illustrates a wireless assembly for use in a blood flow measurement system constructed not in accordance with the principles of the present invention.

FIGS. 10B and 10C are perspective views of the distal end of the wireless assembly of FIG. 10A in a contracted state in FIG. 10B and in a deployed state in FIG. 10C.

FIGS. 11 and 12 depict exemplary graphs showings blood flow measured in accordance with the principles of the present invention.

## VI. Detailed Description Of The Invention

The blood flow measurement system of the present invention comprises devices for measuring and calculating blood flow in a body region, such as the gastrointestinal tract. The devices disclosed herein may utilize a photoplethysmographic approach for measuring the blood flow and, preferably, for measuring perfusion. In accordance with the principles of the present invention, the blood flow measurement system may be optimized for predicting or quickly detecting circulatory, septic, and hypovolemic shock, as well as other situations resulting in hypoperfusion of the gastrointestinal tract. The blood flow measurement may include measurements indicating the amount of red blood cells carrying oxygen in the blood to ensure tissue oxygenation and prevent ischemia resulting in tissue/organ damage.

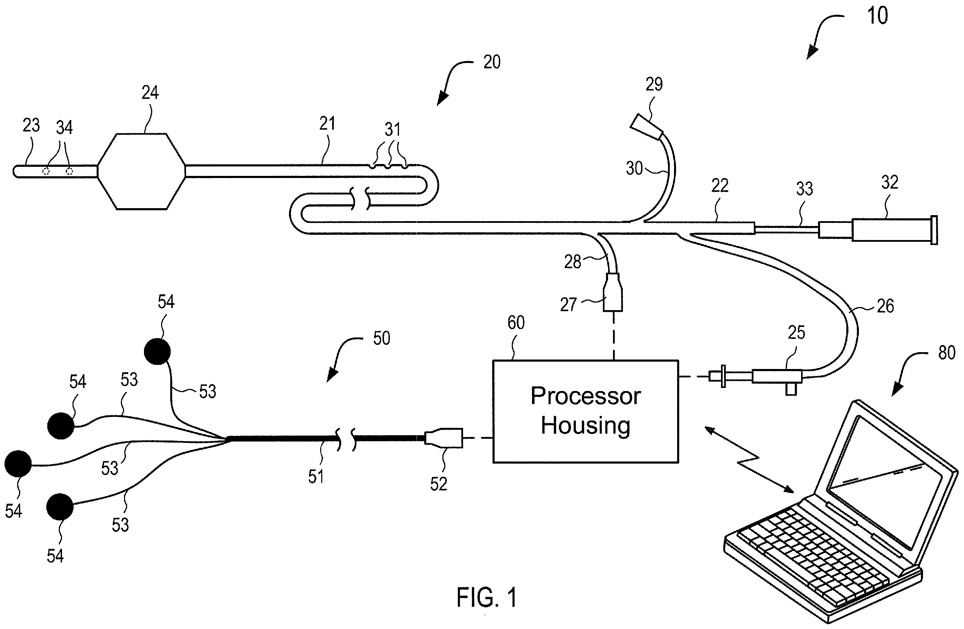

Referring to FIG. 1, an overview of blood measurement system 10 of the present invention is provided. In FIG. 1, components of the system are not depicted to scale on either a relative or absolute basis. Blood measurement system 10 comprises catheter 20, electrocardiogram (ECG) lead assembly 50, processor housing 60, and software-based measurement system 80. In the illustrated embodiment, measurement system 80 is installed and run on a conventional laptop computer used by a clinician or hospital. Processor housing 60 may be coupled, either wirelessly or using a cable, to measurement system 80 such that measurement system 80 may receive and transmit data to processor housing 60. In a preferred embodiment, measurement system 80 is configured to calculate a blood flow rate in the patient's gastrointestinal tract, such as at the capillary bed of the intestine lining, using data received from processor housing 60 as described in further detail below. In one embodiment, measurement system 80 is further configured to calculate a rate of perfusion within the gastrointestinal tract. As used herein, "perfusion" is defined as blood flow to a capillary bed.

Catheter 20 may include shaft 21, proximal end 22, distal end 23, expandable member 24, inflation connector 25 on port 26, electrical connector 27 on port 28, sump connector 29 on sump port 30, sump holes 31, feeding connector 32 on feeding port 33, and feeding holes 34. Shaft 21 comprises a biocompatible tube and may be approximately 115-125 cm in length and preferably 120 cm.

Expandable member 24 may be disposed near distal end 23 and is configured to inflate to expand and to deflate to contract. Expandable member 24 may comprise a suitable biocompatible material as is known in the art and may be a conventional compliant or semi-compliant balloon known in the art of balloon catheters. Expandable member 24 is coupled to inflation connector 25 through port 26 and through a lumen within shaft 21. Inflation connector 25 may be any connector suitable for connection to processor housing 60 for delivering a gas, e.g., air or carbon dioxide, to inflate expandable member 24. As explained in further detail below, a sensor(s) may be disposed within or outside expandable member 24 for sensing predetermined characteristics within the gastrointestinal tract such as blood flow, pressure, and/or impedance.

Electrical connector 27 is coupled via port 28 to the sensor(s) via electrical cables disposed within a lumen of shaft 21. While electrical connector 27 is illustratively shown connected directly to processor housing 60, it should be understood that an extension cable may be used to electrically couple the cables within electrical connector 27 to a processor in processor housing 60. As described in further detail below, the electrical cables may be used to transmit a signal(s) indicative of sensed characteristics within the gastrointestinal tract such as blood flow rate, pressure, and/or impedance.

Sump connector 29 is coupled via sump port 30 to sump holes 31 through a lumen within shaft 21. Sump holes 31 are disposed on shaft 21 to facilitate placement of the holes within the stomach. In one embodiment, the distal-most sump hole 31 is approximately 45-55 cm from the distal tip of catheter 20 and preferably 50 cm. Sump holes 31 are configured to vent gas and/or liquid from the stomach, and sump connector 29 may be any connector suitable for connection to a container (not shown) for receiving the vented gas and/or liquid.

Feeding connector 32 is coupled via feeding port 33 to feeding holes 34 through a lumen within shaft 21. Feeding holes 34 are disposed on shaft 21 and may be distal to expandable member 24. In one embodiment, the distal-most feeding hole 34 is approximately 1-2 cm from the distal tip of catheter 20 and preferably 1.5 cm. Feeding holes 34 are configured to allow suitable food to be released into the duodenum for feeding the patient. Feeding connector 32 may be any connector suitable for connection to a suitable container (not shown) having the food disposed therein.

ECG lead assembly 50 may include main lead 51, ECG connector 52, plurality of leads 53, and plurality of electrode connectors 54. ECG lead assembly 50 is configured to sense an ECG signal based on electrical activity of the patient's heart sensed by electrodes on electrode connectors 54, and may be a conventional ECG lead assembly known to one of ordinary skill in the art. Leads 53 are each independently separable from main lead 51 to facilitate placement of a respective electrode connector 54 at a predetermined body location. While ECG lead assembly 50 illustratively includes four leads 53 and four electrode connectors 54, the scope of the present invention is not limited thereto as would be understood to one of ordinary skill in the art. ECG connector 52 is a suitable connector configured connection to an electrical port or to an electrical cable. While ECG connector 52 is illustratively connected directly to processor housing 60, it should be understood that an extension cable may be used to electrically couple ECG lead assembly 50 to a processor in processor housing 60.

Processor housing 60 is configured to house the control circuitry as well as the pump components for expanding and contracting the expandable member. As described in further detail below, the control circuitry is coupled to the sensor(s) and pump components, and includes memory for storing information from the sensor(s). Processor housing 60 also preferably includes a data port, such as a USB port, that permits the processor to be coupled to measurement system 80 at a hospital or physician's office. Alternatively, processor housing 60 may include a wireless chip, e.g., conforming to the Bluetooth or IEEE 802.11 wireless standards, thereby enabling the processor to communicate wirelessly with measurement system 80.

Measurement system 80 is intended primarily for use by the clinician and comprises software configured to run on a conventional laptop or desktop computer that provides a user interface to components within processor housing 60. The software enables the clinician to configure, monitor, and control operation of catheter 20, ECG lead assembly 50, and control circuitry and pump components within processor housing 60. As described in further detail below, the software may be configured to process a signal indicative of blood flow within a gastrointestinal tract to calculate an area indicative of a blood flow rate within the gastrointestinal tract. In a preferred embodiment, measurement system 80 is configured to allow a clinician to set initial parameters for controlling components within processor housing 60 and for starting and stopping measurements, and the components within processor housing 60 are configured to automatically run after measurement begins without the need for clinician intervention.

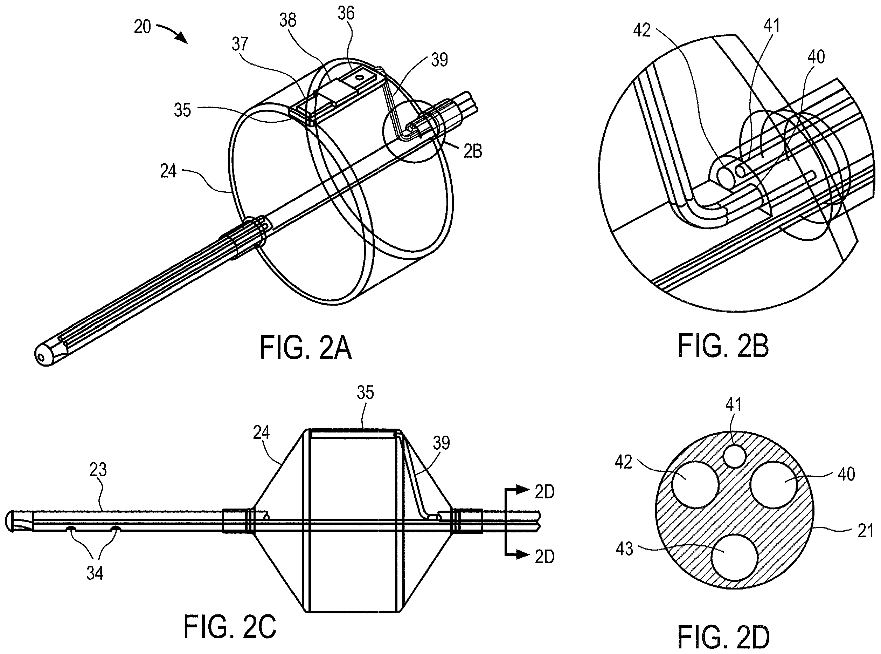

Referring now to FIGS. 2A through 2D, exemplary catheter 20 constructed in accordance with the principles of the present invention is described. Catheter 20 includes expandable member 24 having optical sensor 35 disposed therein. While optical sensor 35 is illustratively disposed within expandable member 24, it should be understood that optical sensor 35 may be disposed outside expandable member 24. Optical sensor 35 may be configured to generate a signal indicative of blood flow, e.g., perfusion, within a gastrointestinal tract of a patient and may include diode 36, photodiode 37, and optional barrier 38. Diode 36 is configured to emit light, e.g., non-visible infrared light, at a body portion, e.g., gastrointestinal tract, and may be a light emitting diode (LED). The emitted light is absorbed at the body portion based on the blood volume at the body portion. Photodiode 37 may be a silicon photodiode and is configured to receive backscattered light reflected from the body portion. The backscattered light corresponds with the variation in blood volume. Optional barrier 38 is disposed between diode 36 and photodiode 37 and is configured to minimize optical crosstalk between diode 36 and photodiode 37 by blocking light emitted from diode 36. Optical sensor cable 39 is configured to transmit the signal from optical sensor 35 to control circuitry within processor housing 60.

Catheter 20 may include cable lumen 40, inflation lumen 41, sump lumen 42, and feeding lumen 43. Cable lumen 40 is configured to receive optical sensor cable 39 and may extend within shaft 21 between expandable member 24 and a lumen within port 28 shown in FIG. 1. Inflation lumen 41 is configured to allow gas to pass therethrough and may extend within shaft 21 between expandable member 24 and a lumen within port 26 shown in FIG. 1. Sump lumen 42 is configured to receive liquid and/or gas vented from the stomach and may extend within shaft 21 between sump holes 31 and a lumen within sump port 30 shown in FIG. 1. FIG. 2B illustratively depicts sump lumen 42 as extending to expandable member 24. In this case, as would be understood by one of ordinary skill in the art, sump lump 42 includes a cap disposed distal to sump holes 31 to prevent liquid and/or gas from the stomach from entering expandable member 24. Feeding lumen 43 is configured to receive food and may extend within shaft 21 between feeding holes 34 and a lumen within feeding port 33 shown in FIG. 1.

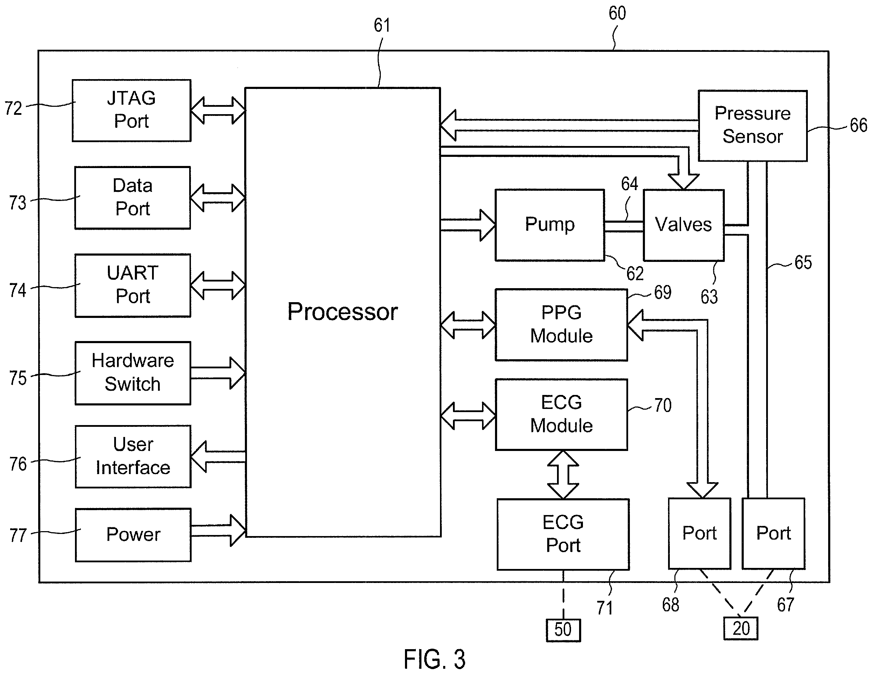

Referring now to FIG. 3, a schematic depicting the functional blocks of components within processor housing 60 of a first embodiment is described. Processor housing 60 illustratively houses control circuitry and pumping components, including processor 61 and pump 62. Pump 62 is configured pump gas, e.g., air or carbon dioxide, to expandable member 24 to periodically inflate and deflate expandable member 24 as directed by processor 61. Pump 62 pumps the gas to valves 63 through line 64. Valves 63 are configured to open to allow the gas to pass therethrough and to close to prevent the gas from passing as directed by processor 61. Gas that passes through valves 63 travels through line 65 to pressure sensor 66 and inflation port 67. Pressure sensor 66 is configured to measure pressure within expandable member 24 and to measure intra-abdominal pressure (IAP) within the patient when expandable member 24 is deflated, and to communicate the measured pressure and the measured IAP to processor 61. Inflation port 67 is configured to couple to inflation connector 25 shown in FIG. 1 to allow gas to pass from pump 62 to expandable member 24.

Processor 61, illustratively the processor of a microcontroller, may include a nonvolatile memory for storing electronic and pump control routines. Processor 61 is electrically coupled to pump 62, valves 63, pressure sensor 66, photoplethysmogram (PPG) module 69, ECG module 70, Joint Test Action Group (JTAG) port 72, data port 73, universal asynchronous receiver/transmitter (UART) port 74, hardware switch 75, user interface 76, and power unit 77. Processor 61 is configured to control electronics and pumping components within processor housing 61 and to transmit data signals a computer having measurement software, e.g., measurement system 80. In one embodiment, processor 61 is a LPC2378 available from NXP Semiconductors of Eindhoven, Netherlands.

PPG module 69 is electrically coupled to electrical port 68. Electrical port 68 is configured to be coupled to electrical connector 27 shown in FIG. 1 to transmit and receive signals from optical sensor 35 shown in FIG. 2. PPG module 69 is configured to generate a PPG signal based on the signal received from optical sensor 35 and to transmit the PPG signal, which may be used to reproduce waveforms produced by pulsating blood, to processor 61. PPG module 69 may be configured to determine blood volume changes based on the signal and the optical properties of tissue and blood at the measuring site. In one embodiment, PPG module 69 is a ChipOx or Pulse Oximetry module available from Corscience of Erlangen, Germany.

ECG module 70 is electrically coupled to ECG port 71. ECG port 71 is configured to be coupled to ECG connector 52 shown in FIG. 1 to transmit and receive signals from ECG lead assembly 50. ECG module 69 is configured to receive an ECG signal from ECG lead assembly 50 and to transmit the ECG signal to processor 61. In one embodiment, ECG module 70 is a EMB1 module available from Corscience of Erlangen, Germany.

JTAG port 72 is any suitable port compliant with JTAG standards and is configured to couple processor 61 to an external processor for debugging and programming processor 61. Data port 73 is any suitable data port, such as a USB port, that permits processor 61 to be coupled to an external computer having measurement software of the present invention loaded thereon. UART port 74 is any suitable UART port configured to connect processor 61 to a network. Additionally, processor housing 60 may include a wireless chip, e.g., conforming to the Bluetooth or IEEE 802.11 wireless standards, thereby enabling processor 61 to communicate wirelessly.

Hardware switch 75 is a suitable switch(es) configured to allow a user to turn components within processor housing 60, including processor 61, on and off. User interface 76 may be a display, preferably an OLED or LCD display, or a plurality of LEDs configured to provide visual confirmation to a user that the components of processor housing are powered and to display suitable messages such as error messages.

Power unit 77 may be a port to allow processor housing 61 to be plugged into a convention 120V wall socket for powering components within the housing. Alternatively, power unit 77 may be a suitable battery such as a replaceable battery or rechargeable battery and processor housing 60 may include circuitry for charging the rechargeable battery, and a detachable power cord.

Referring now to FIGS. 1 through 3, operation of blood measurement system 10 is described. Catheter 20 and ECG lead assembly 50 are operatively coupled to processor housing 60 and processor housing 60 is operatively coupled to measurement system 80. Catheter 20 is orally inserted into a patient through the nose or mouth and into the gastrointestinal tract through the esophagus and stomach. Using fluoroscopic, ultrasonic, anatomic, or CT guidance, expandable member 24 is positioned at a predetermined site within the gastrointestinal tract such as the duodenum. Preferably, catheter 20 is configured such that sump holes 31 are disposed within the stomach when expandable member 24 is disposed within the duodenum. Before, during, or after catheter 20 insertion, electrode connectors 54 of ECG lead assembly 50 may be placed on a suitable site of a patient, such as the patient's chest, to monitor electrical activity of the heart.

After suitable placement of catheter 20 and ECG lead assembly 50, a clinician may input initial parameters into measurement system 80 and may use measurement system 80 to direct processor 61 to begin measurement. Processor 61 then may direct pump 62 to inflate expandable member 24 such that the outer surface of expandable member 24 contacts the inner surface of the gastrointestinal tract site, e.g., intestinal wall of the duodenum. Processor further may initiate processing of signals sensed by ECG lead assembly 50 and ECG module 70 to genereate an ECG signal based on electrical activity of the heart. The ECG signal may be transmitted from ECG lead assembly 50 to and to processor 61. Processor 61 also may direct optical sensor 35 to emit light and receive light reflected from the gastrointestinal tract and to send a signal indicative of blood flow, e.g., perfusion, within the gastrointestinal tract to PPG module 69 based on the reflected light. In one embodiment, the signal is indicative of the blood flow rate corresponding to the gastrointestinal perfusion rate, e.g., blood flow rate at a capillary bed(s) within the intestinal lining. PPG module 69 then generates a PPG signal based on the signal from optical sensor 35 and transmits the PPG signal to processor 61. Processor 61 may further direct pump 62 to periodically deflate and inflate expandable member 24 to continue to monitor blood flow within the gastrointestinal tract. Pressure sensor 66 may monitor pressure within expandable member 24 and intra abdominal pressure within the patient. Advantageously, accurate placement of expandable member 24 and thus feeding holes 34 in the duodenum or post pyloric may be confirmed using the PPG signal and/or the pressure within expandable member 24 when inflated. Processor 61 may transmit data to measurement system 80, e.g., using data port 73 or wirelessly, including data relating to ECG measurement, optical sensor measurement, PPG measurement, and pressure sensor measurement. The data may be used to measure blood flow and/or perfusion within the gastrointestinal tract.

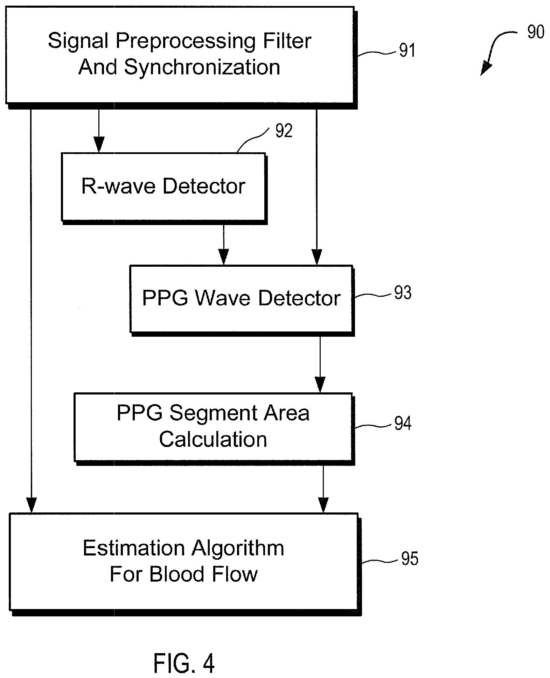

Referring now to FIG. 4, exemplary method 90 for measuring blood flow within the gastrointestinal tract in accordance with the principles of the present invention is described. Method 90 may be performed on a computer having measurement software, e.g., measurement system 80, downloaded thereon or implemented as a program product and stored on a tangible storage device such as machine-readable medium (e.g., tape, compact disk (CD), digital versatile disk (DVD), blu-ray disk (BD), and so forth), external nonvolatile memory device, cloud storage, or other tangible storage medium. In one embodiment, the measurement software comprises customized software. In another embodiment, the measurement software is configured to run on a suitable commercially available software program such as Simulink™ and/or MATLAB™ available from MathWorks, Inc. of Natick, Massachusetts.

At step 91, data is imported into the measurement software based on signals received from processor 61 including the PPG signal and the ECG signal. The data from the PPG signal is filtered using a suitable filter, such as a High-Pass Finite Impulse Response (FIR) filter, to remove noise in the PPG signal, such as noise cause by distortion created by mechanical ventilation or intestine peristalsis. The data then is synchronized to remove filter delays and filter artifacts are removed. The PPG signal and the ECG signal may synchronized relative to time at a suitable frequency, e.g., 100 Hz, using the measurement software.

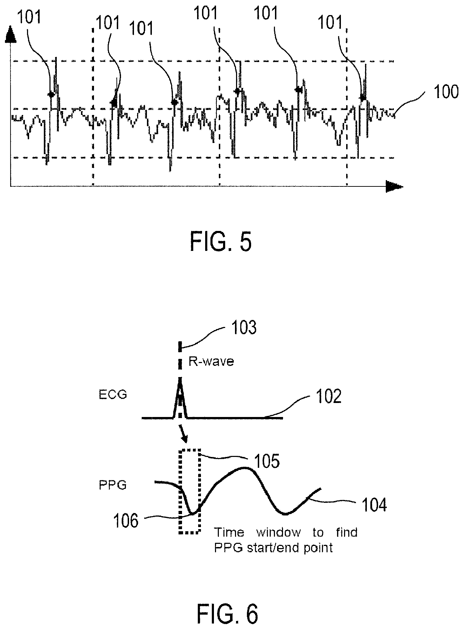

The data based on the ECG signal is used to determine an R-wave signal at step 92. The measurement software may determine the R-wave signal using a derivative based algorithm. Referring to FIG. 5, a graph is illustrated depicting ECG signal 100 having determined R-waves 101 marked thereon. R-waves 101 are used as a trigger to find PPG segments.

Referring back to FIG. 4, the data based on the R-wave signal and the PPG signal are used by the measurement software to determine a PPG segment of the PPG signal at step 93. The PPG signal is searched between two consecutive R-waves and the R-waves are used to determine a time window in which to search for a minimum value of a segment of the PPG signal. The minimum point on the PPG signal within the time window after the R-wave is considered the starting point of PPG segment. The ending point of the PPG segment is where the next minimum point of the PPG signal is detected, which is also a starting point for the next PPG segment. In one embodiment, the time between the starting point and the ending point is the time between pulse beats.

Referring now to FIG. 6, exemplary ECG signal 102 and PPG signal 104 for determining a starting point of a PPG segment are described. ECG signal 102 includes R-wave 103, determined as described above with respect to step 92. Time window 105 is generated after R-wave 103 on PPG signal 104. The measurement software is configured to determine minimum point 106 on PPG signal 104. Minimum point 106 is considered the starting point of a PPG segment on PPG signal 104.

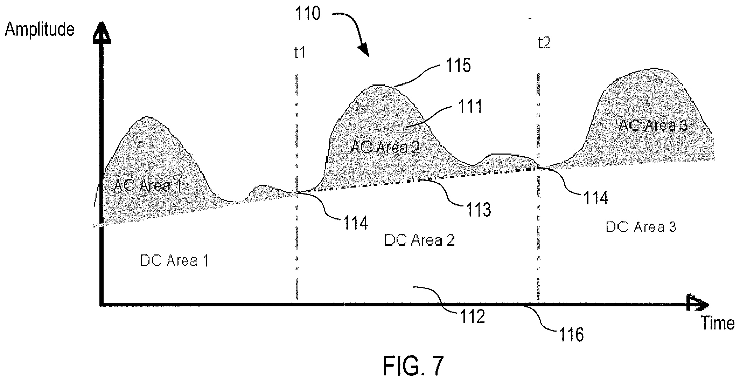

Referring again to FIG. 4, an area is calculated based on the PPG segment using the measurement software in step 94. The area may include an AC area representative of gut perfusion and a DC area representative of blood volume at the gastrointestinal tract site. Referring to FIG. 7, calculation of AC area 111 and DC area 112 of PPG segment 110 is described. To calculate AC area 111, boundary line 113 is drawn between two minimum points 114 on PPG segment 110. AC area 111 then is calculated as the area of PPG segment 110 below PPG signal 115 and above boundary line 113 and between minimum points 114. DC area 112 may be calculated as the area below boundary line 113 and above zero amplitude line 116 and between minimum points 114.

To avoid any bias introduced by heart beat variability, a normalized area may be calculated based on the area for the PPG segment by dividing the area by the time between the starting point and the ending point. In FIG. 7, a normalized area of AC area 111 may be calculated by dividing the AC area by the time between minimum points 114, illustratively:

$$AC\ Area\ 2\ Normalized = (AC\ Area\ 2)/(t2 - t1)$$

Additionally, a normalized area of DC area 112 may be calculated by dividing the DC area by the time between minimum points 114, illustratively:

$$DC\ Area\ 2\ Normalized = (DC\ Area\ 2)/(t2 - t1)$$

Referring back to FIG. 4, in step 95, the blood flow rate and/or perfusion rate within the gastrointestinal tract are measured based on the area calculated for each PPG segment using the measurement software. As described above, the area may include the AC area and/or the DC area. Additional parameters may be used together with the area to calculate the blood flow and/or perfusion rates including the internal balloon pressure as measured by pressure sensor 66, Sp02 value measured at PPG module 69, and/or a ventilator signal transmitted from a conventional ventilator system coupled to the patient and operatively coupled to the measurement software. The measured blood flow and/or perfusion may be displayed numerically and/or graphically on a suitable display, such as a display on the computer running the measurement software, or print-out.

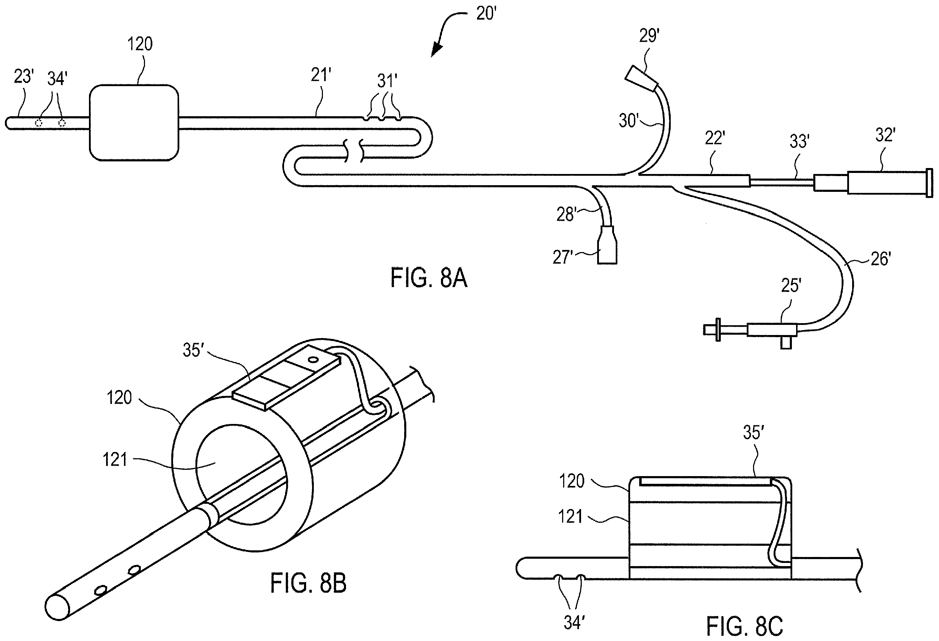

Referring now to FIGS. 8A through 8C, alternative catheter 20' for use in a blood flow measurement system constructed not in accordance with the principles of the present invention is described. Catheter 20' may be substituted for catheter 20 of blood measurement system 10 of FIG. 1. Catheter 20' is constructed substantially identically to catheter 20 of FIG. 1, wherein like components are identified by like-primed reference numbers. Thus, for example, shaft 21' in FIG. 8A corresponds to shaft 21 of FIG. 1, etc. As will be observed by comparing FIGS. 1 and 8A through 8C, various expandable members may be disposed at distal end 23 of catheter 20. For example, expandable member 24 is disposed near distal end 23 of catheter 20 in FIG. 1. However, in FIGS. 8A through 8C, expandable member 120 is disposed near distal end 23' of catheter 20'. Expandable member 120 is a tube-shaped balloon having aperture 121 extending therethrough. Aperture 121 is configured to allow gastric fluid to pass therethrough when catheter 20' is deployed within the gastrointestinal tract and expandable member 120 is expanded/inflated. Advantageously, catheter 20' allows for extended expansion of expandable member 120 without the need to periodically contract/deflate expandable member 120.

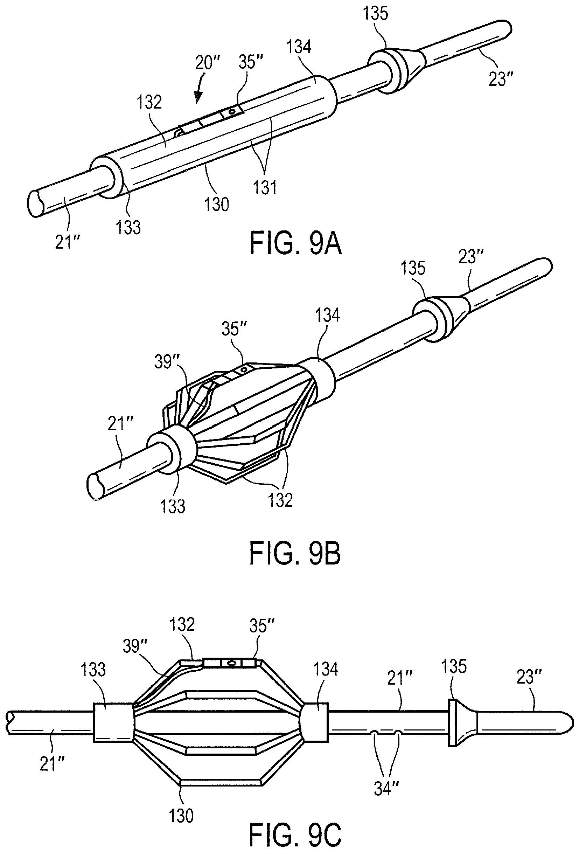

Referring now to FIGS. 9A through 9C, another alternative catheter 20" for use in a blood flow measurement system constructed not in accordance with the principles of the present invention is described. Catheter 20" may be substituted for catheter 20 of blood measurement system 10 of FIG. 1. Catheter 20" is constructed substantially identically to catheter 20 of FIG. 1, wherein like components are identified by like-primed reference numbers. Thus, for example, shaft 21" in FIGS 9A through 9C corresponds to shaft 21 of FIG. 1, etc. As will be observed by comparing FIGS. 1 and 9A through 9C, various expandable members may be disposed at distal end 23 of catheter 20.

Expandable member 130 is disposed on shaft 21" and comprises a plurality of through-wall longitudinal slits 131 defining struts 132. Illustratively, optical sensor 35" is disposed on an outer surface of strut 132, although optical sensor 35" may be disposed on an inner surface of strut 132 and strut 132 may be sufficiently transparent to allow light emitted from optical sensor and reflected from the gastrointestinal tract to pass therethrough. In one embodiment, catheter 20" includes a balloon disposed within expandable member 130 configured to inflate to expand expandable member 130 and deflate to contract expandable member 130. In another embodiment, expandable member 130 comprises a shape-memory alloy, such as nitinol, which has been processed to assume an expanded, deployed state when ejected from a delivery sheath (not shown). In this embodiment, as will be apparent to one of ordinary skill in the art, the blood measurement system would not require inflation components, such as pumps, valves, inflation ports and lumens, etc. Preferably, expandable member 130 is configured and sized such that optical sensor 35" and/or the outer surface of expandable member 130 contact the inner surface of the gastrointestinal tract site, e.g., intestinal wall of the duodenum, when expanded. As depicted in FIGS. 9A through 9C, expandable member 130 illustratively includes a plurality of longitudinal slits disposed circumferentially around expandable member 130 to define a plurality of self-expanding struts 132. The non-slitted proximal portion 133 and distal portion 134 form capture rings. Illustratively, expandable member 130 includes nine slits defining ten self-expanding struts.

In one embodiment, expandable member 130 is fixed on shaft 21". In an alternative embodiment, proximal portion 133 and distal portion 134 permit shaft 21" to freely translate and rotate relative to expandable member 130, without disturbing the location of optical sensor 35" within the gastrointestinal tract. In this embodiment, shaft 21" includes distal stop 135 against which capture ring 134 abuts to limit distal movement of the filter along shaft 21", and optionally may include a proximal stop (not shown) against which proximal capture ring 133 may abut to limit proximal movement.

Advantageously, because struts 132 and capture rings 133 and 134 may be integrally formed from a single tubular segment, the overall diameter of the expandable member in the contracted delivery diameter may be smaller that obtainable using separately-formed struts. Also, because a portion of struts 132 lie flush against the gastrointestinal tract site when deployed, the struts facilitate self-centering and alignment. In addition, the number of separate parts employed in the design, and thus the assembly time and manufacturing cost of the device, are substantially reduced.

While the embodiment of FIGS. 9 illustratively includes nine slits 131 defining ten struts 132, more or less slits (and thus struts) may used, as will be apparent to one of ordinary skill in the art. Moreover, while in the depicted embodiment the longitudinal slits are spaced equi-distant apart around the circumference of expandable member 130 to form equal-width struts, other arrangements may be desirable for specific applications.

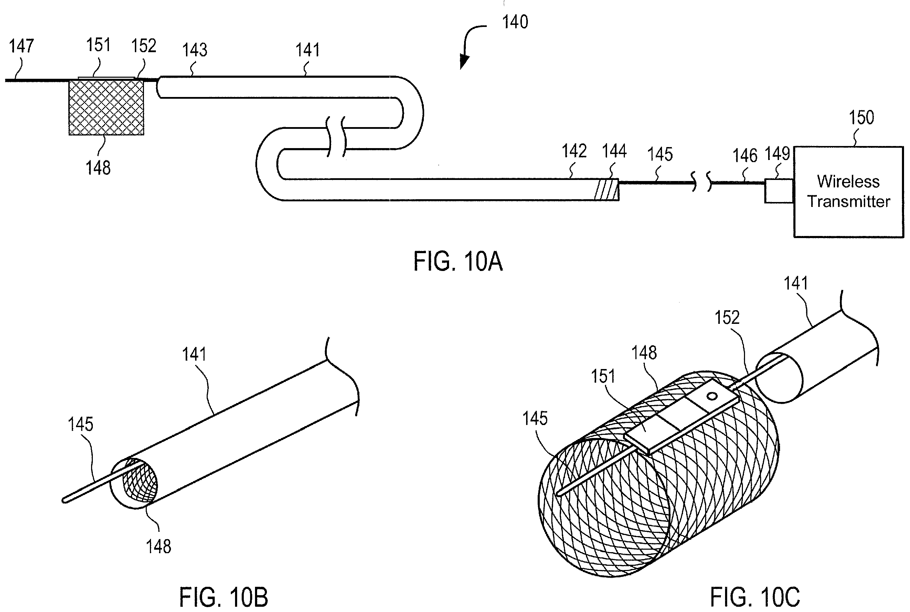

Referring now to FIGS. 10A through 10C, wireless assembly 140 for use in a blood flow measurement system constructed not in accordance with the principles of the present invention is described. Wireless assembly 140 may be substituted for catheter 20 of blood measurement system 10 of FIG. 1. Wireless assembly 140 includes sheath 141 having proximal end 142, distal end 143, and a lumen extending therebetween, wire 145 having proximal end 146 and distal end 147, and wireless transmitter 150. Wire 145 is configured to be disposed within the lumen of sheath 141. Wire 145 further includes expandable member 148, illustratively a stent, optical sensor 141 and optical sensor cable 152. Preferably, expandable member 148 is coupled to wire 145 near distal end 147 and comprises a resilient alloy, such as spring steel or nitinol, which has been processed to transition from a contracted, delivery state within sheath 141 as illustrated in FIG. 10B to an expanded, deployed state as illustrated in FIGS. 10A and 10C when sheath 141 is moved proximally past expandable member 148. In a preferred embodiment, expandable member 148 is configured so that it is capable of expanding and contracting radially to retain optical sensor 151 in apposition to the intestinal wall even when the intestinal diameter fluctuates due to peristalsis. Optical sensor 151 is configured in substantially the same manner as optical sensor 35 and, thus, further description is omitted. Optical sensor cable 152 is configured to transmit the signal from optical sensor 151 to circuitry within wireless transmitter 150. Wireless transmitter 150 is a suitable transmitter that includes a wireless chip, e.g., conforming to the Bluetooth or IEEE 802.11 wireless standards, thereby enabling wireless transmitter 150 to communicate wirelessly. Wireless transmitter 150 is configured to wirelessly transmit the signal from optical sensor 151 to control circuitry within processor housing 60 of FIG. 1. Wireless transmitter may be placed on a suitable site, such as taped to the patient's upper chest.

To secure sheath 141 in place after deployment of expandable member 141, sheath may include threads 144, or other suitable fixation device, disposed at distal end 142 and configured to be screwed into receptacle 149 disposed adjacent to, or optionally within, wireless transmitter 150.

In the embodiments illustrated in FIGS. 10A through 10C, as will be apparent to one of ordinary skill in the art, the blood measurement system would not require inflation components, such as pumps, valves, inflation ports and lumens, etc. and a wireless receiver would be disposed within the processor housing rather than an electrical port. Preferably, expandable member 148 is configured and sized such that optical sensor 151 and/or the outer surface of expandable member 148 contact the inner surface of the gastrointestinal tract site, e.g., intestinal wall of the duodenum, when expanded.

## Examples

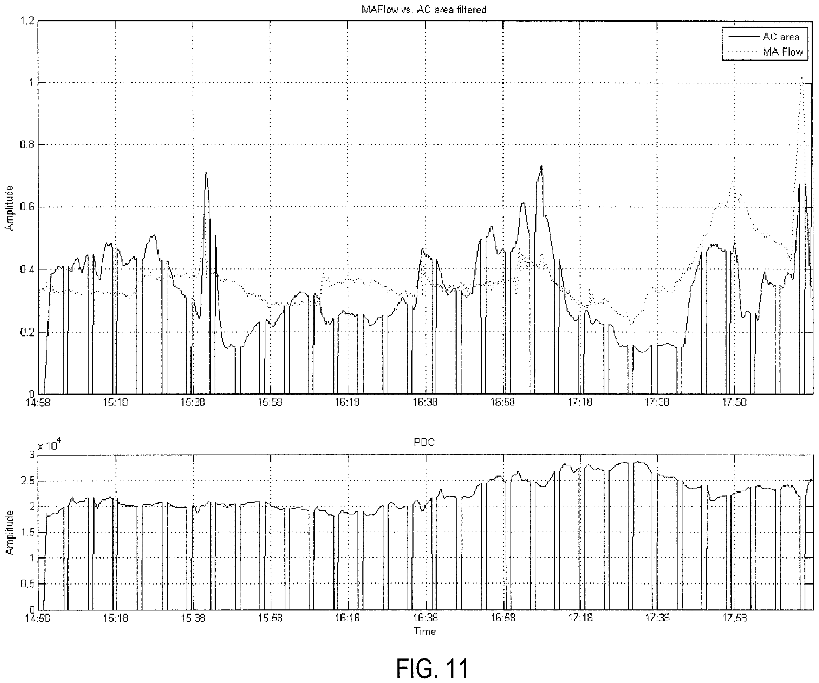

FIG. 11 is a graph comparing mesenteric artery (MA) blood flow of an animal measured by a commercially available apparatus to MA blood flow measured using AC area calculated in accordance with the principles of the present invention. MA flow, illustratively a dotted line, was measured using a Doppler flow probe available from Transonic Systems, Inc of Ithaca, New York. Blood flow measured in accordance with the principles of the present invention is illustratively a solid line. The blood flow was measured for over three hours, during which time the state of the animal was altered by administering various drugs for vasodilation and vasoconstriction, by causing bleeding, and by causing reperfusion of blood. During the three hours, the balloon having the optical sensor disposed therein was deflated every five minutes to allow gastric fluid to pass and to allow for IAP measurement. The deflation of the balloon moved the optical sensor from the intestinal wall which caused a drop in the blood measurement shown by the solid line in FIG. 11. Generally, the blood flow measured using the principles of the present invention correlates well to the blood flow measured using commercially available apparatus.

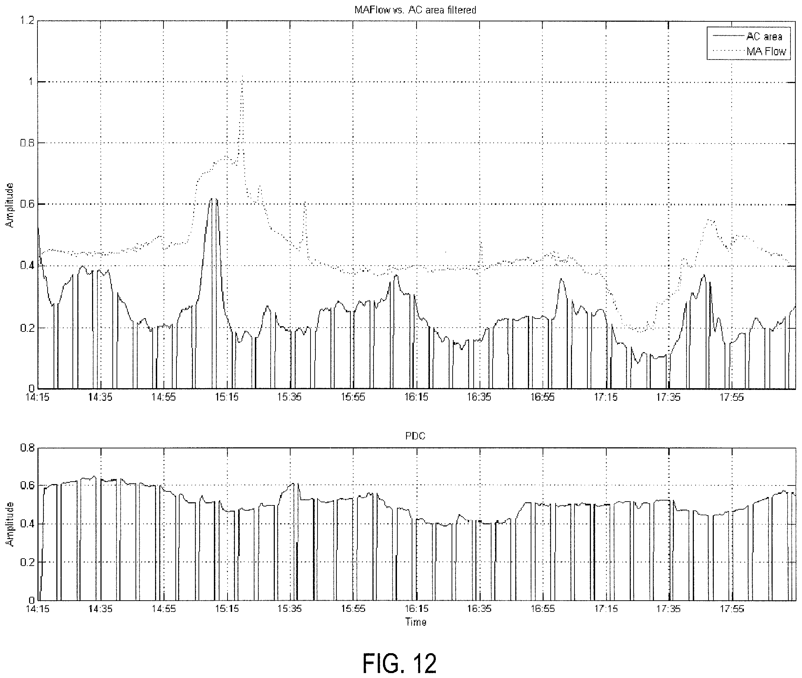

FIG. 12 is a graph comparing MA blood flow of another animal measured by a commercially available apparatus to MA blood flow measured using AC area calculated in accordance with the principles of the present invention. The steps for measurement were similar to the steps described above with respect to FIG. 11, and thus will not be described in detail. Again, generally, the blood flow measured using the principles of the present invention correlates well to the blood flow measured using commercially available apparatus.

While various illustrative embodiments of the invention are described above, it will be apparent to one skilled in the art that various changes and modifications may be made therein without departing from the invention. The appended claims are intended to cover all such changes and modifications that fall within the true scope of the invention.

## Claims

1.  A blood flow measurement system, comprising:

    a catheter configured for placement within a gastrointestinal tract of a patient, the catheter comprising:

        a distal end,
        a proximal end,
        an expandable member disposed near the distal end,
        a lumen extending between the proximal end and the expandable member, and
        an optical sensor disposed adjacent to the expandable member, the optical sensor configured to generate a signal indicative of blood flow within the gastrointestinal tract; and

a processor operatively coupled to the optical sensor and the expandable member, the processor configured to control the optical sensor and to receive the signal, **characterised in that** the processor is further configured to control periodic inflation and deflation of the expandable member.

2. The blood flow measurement system of claim 1, further comprising a pump operatively coupled to the expandable member through the lumen and to the processor, wherein the processor is configured to cause pump to periodically inflate and deflate the expandable member.

3. The blood flow measurement system of claim 2, further comprising a housing configured to house the processor and the pump.

4. The blood flow measurement system of claim 2, further comprising a valve operatively coupled to the pump, the valve configured to control gas flow to the expandable member.

5. The blood flow measurement system of claim 1, further comprising measurement software configured to run on a computer operatively coupled to the processor, the measurement software configured to process the signal to calculate an area indicative of a blood flow rate within the gastrointestinal tract.

6. The blood flow measurement system of claim 5, further comprising a electrocardiogram (ECG) lead assembly operatively coupled to the processor, the ECG lead assembly configured to sense an ECG signal based on electrical activity of a heart of the patient,
wherein the measurement software is further configured to determine an R-wave signal based on the ECG signal.

7. The blood flow measurement system of claim 6, further comprising a photoplethysmogram (PPG) module operatively coupled to the processor and the optical sensor, wherein the PPG module is configured to receive the signal from the optical sensor and to generate a PPG signal based on the signal and to transmit the PPG signal to the processor.

8. The blood flow measurement system of claim 7, wherein the measurement software is further configured to receive the PPG signal and to determine a PPG segment based on the R-wave signal.

9. The blood flow measurement system of claim 8, wherein the measurement software is configured to calculate the area based on the PPG segment for measuring the blood flow rate within the gastrointestinal tract.

10. The blood flow measurement system of claim 1, wherein the optical sensor is disposed within the expandable member.

11. The blood flow measurement system of claim 1, wherein the optical sensor is disposed outside the expandable member.

12. The blood flow measurement system of claim 1, wherein the optical sensor comprises a diode and a photodiode, the diode configured to emit light into the gastrointestinal tract and the photodiode configured to receive the light reflected from the gastrointestinal tract.

13. The blood flow measurement system of claim 1, wherein the signal is indicative of perfusion within the gastrointestinal tract

14. A method for measuring blood flow within a gastrointestinal tract of a patient with a blood flow measurement system according to claim 1, an optical sensor having been introduced into the gastrointestinal tract, the method comprising:

processing a signal indicative of blood flow within the gastrointestinal tract generated using the optical sensor to generate a photoplethysmogram (PPG) signal;
generating an electrocardiogram (ECG) signal based electrical activity of a heart of the patient;
determining a PPG segment of the PPG signal based on the ECG signal; and
measuring blood flow within the gastrointestinal tract based on the PPG segment.

15. The method of claim 14, wherein measuring blood flow comprises measuring perfusion within the gastrointestinal tract based on the PPG segment.

**Patentansprüche**

1. Blutflussmesssystem, umfassend:

    einen Katheter, der konfiguriert ist, um innerhalb eines Gastrointestinaltrakts eines Patienten platziert zu werden, wobei der Katheter Folgendes umfasst:

    ein distales Ende,
    ein proximales Ende,
    ein ausdehnbares Element, das nahe dem distalen Ende angeordnet ist,
    ein Loch, das sich zwischen dem proximalen Ende und dem ausdehnbaren Element erstreckt, und einen optischen Sensor, der benachbart zu dem ausdehnbaren Element angeordnet ist, wobei der optische Sensor konfiguriert ist, um ein Signal zu erzeugen, das den Blutfluss innerhalb des Gastrointestinaltrakts anzeigt; und einen Prozessor, der operativ mit dem optischen Sensor und dem ausdehnbaren Element gekoppelt ist, wobei der Prozessor konfiguriert ist, um den optischen Sensor zu steuern und das Signal zu empfangen, **dadurch gekennzeichnet, dass** der Prozessor ferner konfiguriert ist, um ein periodisches Aufblasen und Entleeren des ausdehnbaren Elements zu steuern.

2. Blutflussmesssystem nach Anspruch 1, umfassend ferner eine Pumpe, die operativ mit dem ausdehnbaren Element durch das Loch hindurch und mit dem Prozessor gekoppelt ist, wobei der Prozessor konfiguriert ist, um zu bewirken, dass die Pumpe das ausdehnbare Element periodisch aufbläst und entleert.

3. Blutflussmesssystem nach Anspruch 2, umfassend ferner ein Gehäuse, das konfiguriert ist, um den Prozessor und die Pumpe aufzunehmen.

4. Blutflussmesssystem nach Anspruch 2, umfassend ferner ein Ventil, das operativ mit der Pumpe gekoppelt ist, wobei das Ventil konfiguriert ist, um den Gasstrom zum ausdehnbaren Element zu steuern.

5. Blutflussmesssystem nach Anspruch 1, umfassend ferner eine Messsoftware, die konfiguriert ist, um auf einem operativ mit dem Prozessor gekoppelten Computer zu laufen, wobei die Messsoftware konfiguriert ist, um das Signal zu verarbeiten, um einen Bereich zu berechnen, der eine Blutflussmenge innerhalb des Gastrointestinaltraktes anzeigt.

6. Blutflussmesssystem nach Anspruch 5, umfassend ferner eine Elektrokardiogramm (ECG)-Leiteranordnung, die operativ mit dem Prozessor gekoppelt ist, wobei die ECG-Leiteranordnung konfiguriert ist, um ein ECG-Signal basierend auf der elektrischen Aktivität eines Herzens des Patienten zu erfassen, bei dem die Messsoftware ferner konfiguriert ist, um ein R-Wellensignal basierend auf dem ECG-Signal zu ermitteln.

7. Blutflussmesssystem nach Anspruch 6, umfassend ferner ein Photoplethysmogramm (PPG)-Modul, das operativ mit dem Prozessor und dem optischen Sensor gekoppelt ist, bei dem das PPG-Modul konfiguriert ist, um das Signal von dem optischen Sensor zu empfangen und ein PPG-Signal basierend auf der Signal zu erzeugen und das PPG-Signal an den Prozessor zu senden.

8. Blutflussmesssystem nach Anspruch 7, bei dem die Messsoftware ferner konfiguriert ist, um das PPG-Signal zu empfangen und um ein PPG-Segment basierend auf dem R-Wellensignal zu ermitteln.

9. Blutflussmesssystem nach Anspruch 8, bei dem die Messsoftware konfiguriert ist, um den Bereich basierend auf dem PPG-Segment zu berechnen, um die Blutflussmenge im Gastrointestinaltrakt zu messen.

10. Blutflussmesssystem nach Anspruch 1, bei dem der optische Sensor innerhalb des ausdehnbaren Elements angeordnet ist.

11. Blutflussmesssystem nach Anspruch 1, bei dem der optische Sensor außerhalb des ausdehnbaren Elements angeordnet ist.

**12.** Blutflussmesssystem nach Anspruch 1, bei dem der optische Sensor eine Diode und eine Photodiode umfasst, wobei die Diode konfiguriert ist, um Licht in den Gastrointestinaltrakt zu senden und die Photodiode konfiguriert ist, um das von dem Gastrointestinaltrakt reflektierte Licht zu empfangen.

**13.** Blutflussmesssystem nach einem der Ansprüche 1 bis 13, bei dem das Signal eine Perfusion im Gastrointestinaltrakt anzeigt.

**14.** Verfahren zum Messen des Blutflusses innerhalb eines Gastrointestinaltraktes eines Patienten mit einem Blutflussmesssystem nach Anspruch 1, wobei ein optischer Sensor in den Gastrointestinaltrakt eingeführt worden ist, wobei das Verfahren Folgendes umfasst:

Verarbeiten eines Signals, das den Blutfluss in dem Gastrointestinaltrakt anzeigt, das unter Verwendung des optischen Sensors erzeugt worden ist, um ein Photoplethysmogramm (PPG)-Signal zu erzeugen;
Erzeugen eines Elektrokardiogramm (ECG)-Signal basierend auf der elektrischen Aktivität eines Herzens des Patienten;
Bestimmen eines PPG-Segments des PPG-Signals basierend auf dem ECG-Signal; und
Messen des Blutflusses im Gastrointestinaltrakt basierend auf dem PPG-Segment.

**15.** Verfahren nach Anspruch 14, bei dem das Messen des Blutflusses das Messen der Perfusion innerhalb des Gastrointestinaltrakts basierend dem PPG-Segment umfasst.

**Revendications**

**1.** Système de mesure du débit sanguin, comprenant:

un cathéter configuré pour être placé dans un tractus gastro-intestinal d'un patient, le cathéter comprenant:

une extrémité distale,
une extrémité proximale,
un élément extensible disposé à proximité de l'extrémité distale,
une lumière s'étendant entre l'extrémité proximale et l'élément extensible, et un capteur optique disposé adjacent à l'élément extensible, le capteur optique étant configuré pour générer un signal indicatif du débit sanguin dans le tractus gastro-intestinal; et un processeur couplé en fonctionnement au capteur optique et à l'élément extensible, le processeur étant configuré pour commander le capteur optique et pour recevoir le signal, **caractérisé par le fait que** le processeur est par ailleurs configuré pour commander le gonflement et dégonflement périodique de l'élément extensible.

**2.** Système de mesure du débit sanguin selon la revendication 1, comprenant par ailleurs une pompe couplée en fonctionnement à l'élément extensible à travers la lumière et au processeur, dans lequel le processeur est configuré pour faire que la pompe gonfle et dégonfle périodiquement l'élément extensible.

**3.** Système de mesure du débit sanguin selon la revendication 2, comprenant par ailleurs un boîtier configuré pour accueillir le processeur et la pompe.

**4.** Système de mesure du débit sanguin selon la revendication 2, comprenant par ailleurs une soupape couplée en fonctionnement à la pompe, la soupape étant configurée pour commander le débit de gaz vers l'élément extensible.

**5.** Système de mesure de débit sanguin selon la revendication 1, comprenant par ailleurs un logiciel de mesure configuré pour être exécuté sur un ordinateur couplé en fonctionnement au processeur, le logiciel de mesure étant configuré pour traiter le signal pour calculer une zone indicative d'un débit sanguin dans le tractus gastro-intestinal.

**6.** Système de mesure du débit sanguin selon la revendication 5, comprenant par ailleurs un ensemble de conducteurs d'électrocardiogramme (ECG) couplé en fonctionnement au processeur, l'ensemble de conducteurs ECG étant configuré pour détecter un signal d'ECG sur base de l'activité électrique d'un coeur du patient, dans lequel le logiciel de mesure est par ailleurs configuré pour déterminer un signal d'onde R sur base du signal d'ECG.

**7.** Système de mesure de débit sanguin selon la revendication 6, comprenant par ailleurs un module de photopléthysmogramme (PPG) couplé en fonctionnement au processeur et au capteur optique, dans lequel le module de PPG est configuré pour recevoir le signal du capteur optique et pour générer un signal de PPG sur base du signal et pour transmettre le signal de PPG au processeur.

8.  Système de mesure de débit sanguin selon la revendication 7, dans lequel le logiciel de mesure est par ailleurs configuré pour recevoir le signal de PPG et pour déterminer un segment de PPG sur base du signal d'onde R.

9.  Système de mesure du débit sanguin selon la revendication 8, dans lequel le logiciel de mesure est configuré pour calculer la zone sur base du segment de PPG pour mesurer le débit sanguin dans le tractus gastro-intestinal.

10. Système de mesure du débit sanguin selon la revendication 1, dans lequel le capteur optique est disposé à l'intérieur de l'élément extensible.

11. Système de mesure du débit sanguin selon la revendication 1, dans lequel le capteur optique est disposé à l'extérieur de l'élément extensible.

12. Système de mesure de débit sanguin selon la revendication 1, dans lequel le capteur optique comprend une diode et une photodiode, la diode étant configurée pour émettre de la lumière dans le tractus gastro-intestinal et la photodiode étant configurée pour recevoir la lumière réfléchie par le tractus gastro-intestinal.

13. Système de mesure du débit sanguin selon la revendication 1, dans lequel le signal est indicatif de perfusion dans le tractus gastro-intestinal.

14. Procédé pour mesurer le flux sanguin dans un tractus gastro-intestinal d'un patient par un système de mesure du débit sanguin selon la revendication 1, un capteur optique ayant été introduit dans le tractus gastro-intestinal, le procédé comprenant le fait de:

> traiter un signal indicatif du flux sanguin dans le tractus gastro-intestinal généré à l'aide du capteur optique pour générer un signal de photopléthysmogramme (PPG);
> générer une activité électrique à base de signal d'électrocardiogramme (ECG) d'un coeur du patient;
> déterminer un segment de PPG du signal PPG sur base du signal d'ECG; et
> mesurer le débit sanguin dans le tractus gastro-intestinal sur base du segment de PPG.

15. Procédé selon la revendication 14, dans lequel la mesure du flux sanguin comprend le fait de mesurer la perfusion dans le tractus gastro-intestinal sur base du segment de PPG.

FIG. 1

FIG. 2A

FIG. 2B

FIG. 2C

FIG. 2D

EP 2 819 573 B1

FIG. 3

EP 2 819 573 B1

90

91 Signal Preprocessing Filter And Synchronization

92 R-wave Detector

93 PPG Wave Detector

94 PPG Segment Area Calculation

95 Estimation Algorithm For Blood Flow

FIG. 4

FIG. 5

FIG. 6

EP 2 819 573 B1

FIG. 7

EP 2 819 573 B1

FIG. 8A

FIG. 8B

FIG. 8C

FIG. 9A

FIG. 9B

FIG. 9C

FIG. 10A

FIG. 10B

FIG. 10C

EP 2 819 573 B1

FIG. 11

FIG. 12

**EP 2 819 573 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080319339 A, Beute
- US 7618376 B, Kimball
- US 5743261 A, Mainiero